Europäisches Patentamt

European Patent Office

Office européen des brevets

(11) Veröffentlichungsnummer: **0 272 336 B1**

## (12) EUROPÄISCHE PATENTSCHRIFT

(45) Veröffentlichungstag der Patentschrift: **23.10.91**

(51) Int. Cl.⁵: **A61K 47/00, A61K 31/44**

(21) Anmeldenummer: **86117660.0**

(22) Anmeldetag: **18.12.86**

(54) **Gegenüber Lichteinfluss stabilisiertes Nifedipin-Konzentrat und Verfahren zu seiner Herstellung.**

(43) Veröffentlichungstag der Anmeldung:
**29.06.88 Patentblatt 88/26**

(45) Bekanntmachung des Hinweises auf die
Patenterteilung:
**23.10.91 Patentblatt 91/43**

(84) Benannte Vertragsstaaten:
**AT BE CH DE ES FR GB GR IT LI LU NL SE**

(56) Entgegenhaltungen:
**EP-A- 0 182 007**
**LU-A- 65 929**

**CHEMICAL ABSTRACTS, Band 66, Nr. 23, 5.**
**Juni 1967, Seite 10124, Zusammenfassung**
**Nr. 108175n, Columbus, Ohio, US; V. DAS**
**GUPTA: "Evaluation of sunscreen agents", &**
**J. SOC. COSMET. CHEM. 18(3), 143-7(1967)**

(73) Patentinhaber: **Bauer, Kurt H. Prof. Dr.**
**Im Finkeler 4**
**W-7800 Freiburg33(DE)**

(72) Erfinder: **Bauer, Kurt H. Prof. Dr.**
**Im Finkeler 4**
**W-7800 Freiburg33(DE)**

(74) Vertreter: **Kraus, Walter, Dr. et al**
**Patentanwälte Kraus, Weisert & Partner**
**Thomas-Wimmer-Ring 15**
**W-8000 München 22(DE)**

## Beschreibung

Die Erfindung betrifft ein gegenüber Lichteinfluß stabilisiertes Nifedipin-Konzentrat, ein Verfahren zu seiner Herstellung, ein Arzneimittel, die dieses enthält, und seine Verwendung.

Nifedipin ist ein sehr schwer löslicher, nicht dissoziierender Ca-Antagonist, der zudem außerordentlich lichtempfindlich ist. Nifedipin ist in Wasser nur etwa 1:200.000 löslich, in Ethanol und in Glycerol ist es wenig, in Chloroform und Aceton besser löslich.

Aufgrund der Lichtempfindlichkeit und der schweren Löslichkeit von Nifedipin treten bei der galenischen Zubereitung von Arzneimittelspezialitäten eine Reihe von Schwierigkeiten auf, wie aus zahlreichen Patenten und Patentanmeldungen für spezielle Formulierungen dieses Wirkstoffs ersichtlich wird.

Die Empfindlichkeit von Nifedipin gegenüber Tageslicht und UV-Strahlen kann am besten durch die Halbwertszeit einer Nifedipinlösung am Tageslicht zum Ausdruck gebracht werden, die nur wenige Minuten beträgt (K. Thoma et al., PHARM.IND. 47, 207-215, 319-327 (1985)). Diese Lichtempfindlichkeit ist bedeutend stärker als die der meisten bekannten Pharmazeutika. Nifedipin kann deshalb nur bei völliger Dunkelheit, zumindest aber unter Rot- und Gelblicht, bearbeitet und zu Arzneimittelzubereitungen verarbeitet werden.

Wegen der schlechten Löslichkeit wird Nifedipin bis jetzt überwiegend in lösliche, hydrophile Polymere, wie Polyethylenglykole, Polyvinylpyrrolidon oder Celluloseether, eingebettet. Dabei entstehen feste Lösungen, wenn es sich in der Schmelze, z.B. von Polyethylenglykol löst, oder Co-Präzipitate, wenn es z.B. in Polyvinylpyrrolidon mit geeigneten Lösungsmitteln gelöst und durch Entfernung der Lösungsmittel feinstverteilt ausgefällt wird.

Die bekannten festen Lösungen in Polyethylengykol enthalten mindestens die dreifache Menge Polyethylenglykol. Diesen festen Lösungen und auch den Co-Präzipitaten können erforderlichenfalls noch Tenside oder ähnliche Verbindungen zugesetzt werden.

In der DE-A 3 438 830 wird eine Nifedipin als Wirkstoff enthaltende Darreichungsform beschrieben, in der das Nifedipin molekulardispers in Form einer erstarrten Schmelze in einer Mischung aus flüssigen und nichtflüssigen Polyethylenglykolen vorliegt, in Form der Schmelze in Gelatine-Hart-Kapseln abgefüllt wird. Zur Verbesserung der Stabilität wird der Farbstoff "Gelborange S" verwendet. Die Verwendung von Gelborange S als Farbstoff ist bedenklich, da Gelborange S ein Azofarbstoff ist.

Die außerordentlich hohe Lichtempfindlichkeit wird dadurch berücksichtigt, daß in der Dunkelheit oder unter Rot-bzw. Gelblicht gearbeitet wird. Außerdem werden Zubereitungen, die den Arzneistoff Nifedipin enthalten, in Gelatinekapseln abgefüllt oder mit Filmumhüllungen umgeben, deren Hüllen zum Zwecke der Lichtfiltration mit Gelborange S, Food-Yellow No. 4 oder 5, oder mit Eisenoxidfarben (z.B. E 110) und Titandioxid eingefärbt werden. Daneben ist auch noch die Einarbeitung von UV-Absorbern, wie Phenylsalicylat, gebräuchlich. Auch Verpackungsmaterialien können mit geeigneten Farbstoffen eingefärbt werden.

Die hohe Lichtempfindlichkeit und die schwere Löslichkeit von Nifedipin bedingen aufwendige Verfahrensmaßnahmen bei der Herstellung von Arzneimittelzubereitungen.

Der vorliegenden Erfindung liegt die Aufgabe zugrunde, den Nachteil der bisher bekannten Nifedipin enthaltenden Präparate zu beseitigen und ein Nifedipin-Konzentrat zur Verfügung zu stellen, mit dem es möglich ist, Nifedipin ohne große Mengen an Zusatzstoffen zu Arzneimitteln verarbeiten zu können, aus denen es rasch und in ausreichender Konzentration freigesetzt wird. Das erfindungsgemäße Nifedipin-Konzentrat soll weiterhin eine bessere Lichtbeständigkeit aufweisen als Nifedipin selbst. Erfindungsgemäß soll ein Verfahren zur Herstellung von Nifedipin enthaltenden Arzneimitteln zur Verfügung gestellt werden, gemäß dem das relativ schlecht lösliche Nifedipin ausreichend fein dispers in Matrices (Einbettungen) eingearbeitet werden kann, so daß die Arzneistofffreisetzung bzw. die Auflösungsgeschwindigkeit verbessert wird. Erfindungsgemäß soll die feinstdispergierte Einarbeitung des außergewöhnlich schlecht löslichen Nifedipins, sei es molekulardispers in gelöster Form oder suspendiert bzw. dispergiert (nicht gelöst) in möglichst kleinen agglomerierten Molekülverbänden möglich sein, um eine rasche und ausreichende Freigabe (Auflösungsgeschwindigkeit) und schließlich eine optimale Resorption zu gewährleisten.

Erfindungsgemäß soll eine Verbesserung des Lichtschutzes durch möglichst harmlose Zuschläge, zum Beispiel durch Zusatz eines Vitamins erfolgen.

Gegenstand der Erfindung ist ein gegenüber Lichteinfluß stabilisiertes Nifedipin-Konzentrat, das dadurch gekennzeichnet ist, daß es Nifedipin, Polyethylenglykol, 0,5 bis 20 Gew.-% Vitamin $B_2$, bezogen auf das Nifedipin, mindestens ein Tensid und gegebenenfalls übliche Trägerstoffe enthält.

Durch die erfindungsgemäß ausgewählten und eingearbeiteten Tenside gelingt es Nifedipin in hohem Maße auch in höherer Konzentration in Lösung zu bringen. Der nicht mehr gelöste Anteil wird durch die Tenside derart fein dispergiert, daß er sich fast wie gelöstes Nifedipin verhält.

Im Falle des Vitamins $B_2$ mit Nifedipin tritt die Problematik der unterschiedlichen Löslichkeiten und der Stabilität auf. In der Vergangenheit wurden entweder Farbstoffe, andere Wirkstoffe oder UV-Absorbentien verwendet. Überraschenderweise wurde gefunden, daß Vitamin $B_2$ eine gute Lichtschutzwirkung für Nifedipin aufweist. Die physikalische Unverträglichkeit, die aufgrund der unterschiedlichen Löslichkeiten von Nifedipin und Vitamin $B_2$ auftritt, konnte überraschenderweise durch das ausgewogene Zusammenwirken von PEG, organischen Lösungsmitteln, einem geeigneten Tensid und der erforderlichen Menge Wasser behoben werden.

In der Vergangenheit wurden die festen Lösungen oder Präzipitate mit Nifedipin im Verhältnis von mindestens 1:3 hergestellt. Die Mischung Polyethylenglykol und die angegebenen Tenside gestatten höhere Konzentrationen von Nifedipin bei vergleichbarer Auflösungsgeschwindigkeit bzw. Bioverfügbarkeit.

Die Erfindung betrifft weiterhin ein Verfahren zur Herstellung der gegenüber Lichteinfluß stabilisierten Nifedipin-Konzentrate, das dadurch gekennzeichnet ist, daß man Nifedipin, das Polyethylenglykol und gegebenenfalls das Tensid in einem organischen Lösungsmittel, gegebenenfalls unter Erwärmen, löst und zu der nifedipinhaltigen Lösung eine wäßrige Lösung oder Suspension von Vitamin $B_2$ und gegebenenfalls Tensid zugibt, wobei mindestens eine der beiden Lösungen bzw. die Suspension Tensid enhält, die beiden Lösungen mischt und in an sich bekannter Weise eindampft und das stabilisierte Einbettungsprodukt gewinnt und gegebenenfalls granuliert.

Die Schwierigkeit bei der Herstellung der erfindungsgemäßen Arzneimittelzubereitung liegt darin, daß Nifedipin nur in bestimmten organischen Lösungsmitteln löslich ist, Vitamin $B_2$ dagegen nur in Wasser löslich ist. Deshalb muß das Vitamin $B_2$ zuerst in Wasser soweit als möglich gelöst werden und der nicht mehr lösliche Rest suspendiert werden. Zuviel Wasser verbietet sich, weil die Ausfällung von Nifedipin beim Zusammenmischen suspendiert wird und außerdem zulange Trocknungszeiten resultieren. Überraschenderweise zeigte sich, daß durch das Polyethylenglykol und das Tensid die wäßrige Lösung bzw. Suspension ausreichend fein dispergiert.

Die Erfindung betrifft ebenfalls ein Arzneimittel, das Nifedipin, Polyethylenglykol, 0,5 bis 20 Gew.-% Vitamin $B_2$, bezogen auf das Nifedipin, mindestens ein Tensid sowie übliche Arzneimittelträgerstoffe und gegebenenfalls Verdünnungsmittel enthält.

Die Erfindung betrifft außerdem die Verwendung von dem oben erwähnten Nifedipin-Konzentrat zur Herstellung von Arzneimittelzubereitungen.

Überraschenderweise zeigte sich, daß das Nifedipin in Polyethylenglykol unter Zuhilfenahme von geeigneten Lösungsmitteln bereits im Verhältnis 1:1 eingebettet werden kann. Es war weiterhin überraschend, daß durch die Einarbeitung von Vitamin $B_2$ in das Nifedipin ein überraschend guter Lichtschutz erreicht wird.

Erfindungsgemäß kann das Nifedipin unter Zuhilfenahme von geeigneten Lösungsmitteln, z.B. Aceton, chlorierten Kohlenwasserstoffen oder Toluol, die anschließend wieder entfernt werden, bereits im Verhältnis 1:1 in Polyethylenglykol gelöst bzw. eingebettet werden. Das Verhältnis Nifedipin: Polyethylenglykol kann im Bereich zwischen 2:1 und 1:3 liegen.

Die besten Löslichkeiten zeigt Nifedipin in Aceton und verschiedenen bzw. bestimmten chlorierten Kohlenwasserstoffen, und zwar ca. 30%. Weitere Löslichkeiten sind wie folgt:

| | | |
|---|---|---|
| in Alkoholen (Ethanol) | ca. | 2,6% |
| in Tween | ca. | 2% |
| in Solketal | ca. | 3% |
| in 1,2-Propylenglykol | ca. | 64% |
| in Benzoylalkohol | ca. | 11% |
| in PEG 400 | | 6,1% |
| in Ricinusöl | | 0,54% |
| in fl. Paraffin | > | 0,05% |

Durch Einarbeitung von 0,5 bis 20 Gew.-%, vorzugsweise 5 bis 10 Gew.-%, Vitamin $B_2$ (Riboflavin, Lactoflavin), bezogen auf das Nifedipin, wird ein überraschend guter Lichtschutz erreicht. Das Vitamin $B_2$ muß weitgehend in gelöster Form eingearbeitet werden. Dazu sind in der Regel 5 bis 20 Teile Wasser pro 1 Teil Vitamin $B_2$ erforderlich.

Zur Optimierung der Verbindung bzw. der Emulgierung von hydrophilem Vitamin $B_2$ und dem hydrophoben Nifedipin ist der Zusatz von mindestens einem geeigneten Tensid erforderlich. Hierzu sind geeignet Ethoxy-Propoxy-Copolymere (z.B. Pluronics ®, Polyoxyethylen-Fettsäureester oder Polyoxyethylen-Fettalkoholether, ethoxylierte, hydrierte Ricinusölprodukte (z.B. Cremophor RH 60 ® ) oder ähnliche Verbindungen. Man kann auch Gemische von Tensiden verwenden. Das Tensid wird in einer Menge von 2 bis 50 Teilen, vorzugsweise 5 bis 25 Teilen, bezogen auf 50 Teile Nifedipin, eingearbeitet.

Der Tensidzusatz vermittelt nicht nur die Dispergierung zwischen dem hydrophoben Nifedipin und dem hydrophilen Vitamin $B_2$, sondern verbessert außerdem die Freisetzung bzw. die Bioverfügbarkeit des Nifedipins.

Dies ist die Grundlage dafür, daß die Konzentration des Nifedipins in den erfindungsgemäßen Zubereitungen erhöht werden konnte.

Zur Herstellung der erfindungsgemäßen Nifedipin-Konzentrate löst man das Nifedipin und das Polyethylenglykol im Lösungsmittel, gegebenenfalls unter Erwärmen, und gibt hierzu eine Lösung oder Suspension von Vitamin $B_2$ in Wasser.

Die erhaltene Lösung bzw. Dispersion wird dann getrocknet. Die Trocknung kann in an sich bekannter Weise erfolgen, beispielsweise durch Sprühtrocknen oder durch Erstarren auf Kühlwalzen. Läßt man das Produkt auf Kühlwalzen erstarren, so erfolgt nach dem Trocknen eine Zerkleinerung, beispielsweise ein Vermahlen oder ein Granulieren.

Die erfindungsgemäßen Nifedipin-Konzentrate können in an sich bekannter Weise durch Vermischen mit üblichen Arzneimittelträgerstoffen oder auch durch Zusammenschmelzen mit den üblichen Hilfsstoffen zu Pulvermischungen oder Granulaten verarbeitet werden, die als Arzneimittel in an sich bekannter Weise eingesetzt werden können. Die Granulate können beispielsweise in Hartgelatinekapseln abgefüllt werden oder zu Tabletten komprimiert werden. Durch Auflösen in flüssigen Polyethylenglykolen, anderen Glykolen, Glycerin mit Menthol und ähnlichen Hilfsstoffen können aus den Konzentraten auch Weichgelatinekapseln und Tropfen hergestellt werden.

Über die Verbesserung der Lichtstabilität geben die beiliegenden UV-spektralphotometrischen Kurven Auskunft. Nach S. Ebel et al., ARZNEIM.FORSCH. 28 (12), 2188-2193 (1978) und K. Thoma et al., PHARM.IND. 47 (2), 207-215 (1985) verändert sich das UV-Spektrum von Nifedipin während einer Belichtung, z.B. mit Tageslicht, indem sich bei 280 nm ein Peak ausbildet sowie durch Absenken des Plateaus bei ca. 350 nm. Die Verschiebungen bei den beigefügten UV-Kurven zeigen, daß im Vergleich mit den Anfangskurven bei dem mit Riboflavin stabilisierten, eingebetteten Nifedipin nach 3tägiger Belichtung eine Verbesserung der Lichtstabilität um etwa zwei Drittel erreicht wird, d.h. die Instabilität beträgt nur noch rund ein Drittel gegenüber dem nichtstabilisierten Produkt.

Es zeigen:

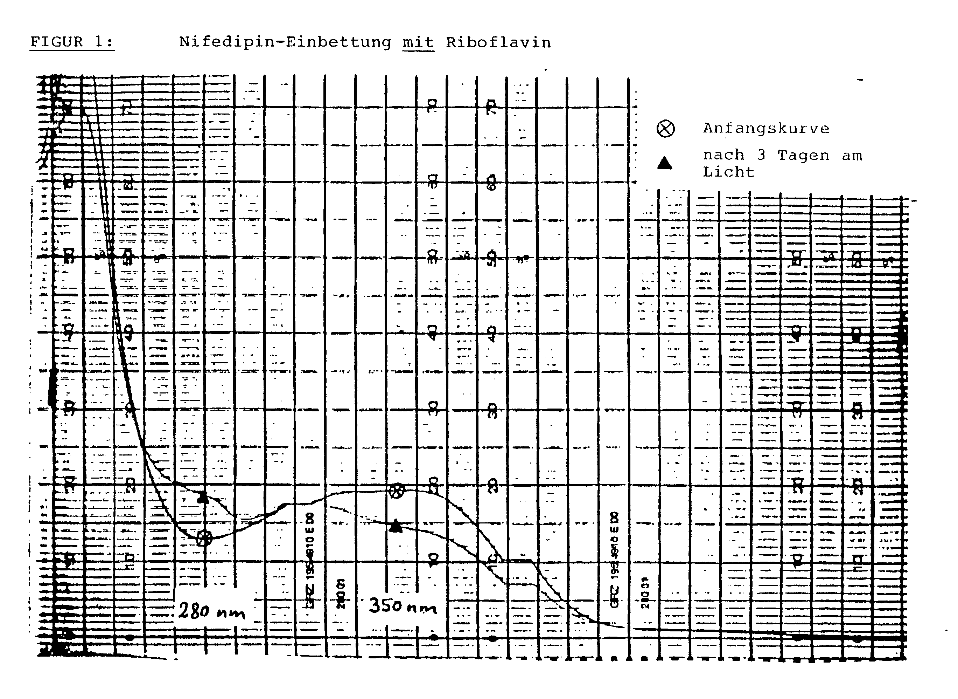

Figur 1    die Nifedipineinbettung Riboflavin,

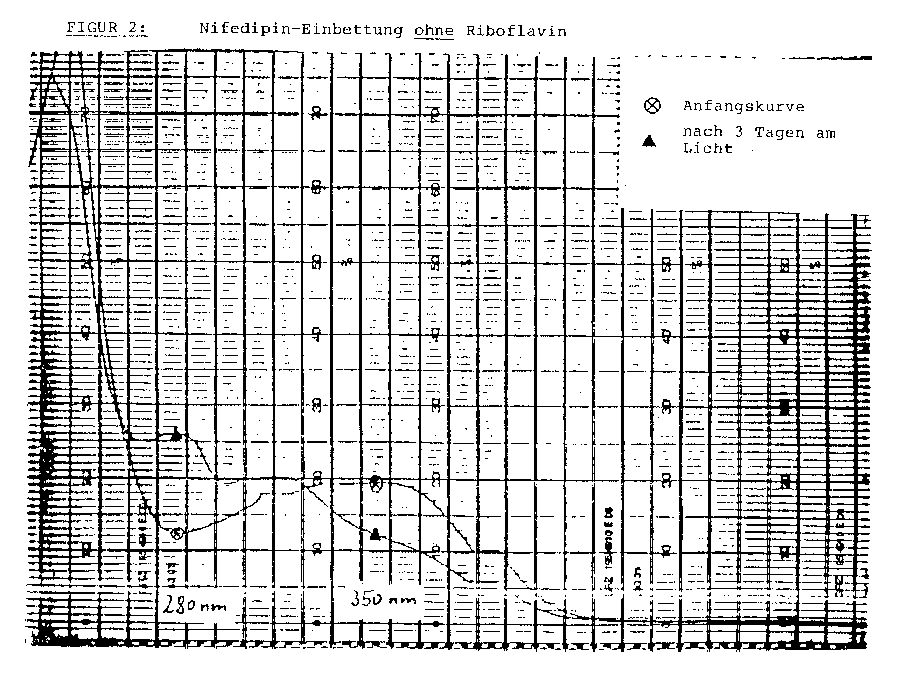

Figur 2    die Nifedipineinbettung ohne Riboflavin.

Der Vorteil dabei ist, daß infolge des feinstdispersen Zusatzes von Vitamin $B_2$ bei den Herstellungsgängen kein absoluter Lichtschutz mehr notwendig ist und auch der Lichtschutz der Endprodukte verbessert ist.

Die folgenden Beispiele erläutern die Erfindung.

**Beispiel 1**

50 Teile Nifedipin werden in der warmen Lösung (ca.60-80 °C) von 10 Teilen Poloxamer 188® und 35 Teilen Polyethylenglykol 6000 in 60 bis 80 Teilen Toluol gelöst.

Getrennt werden 5 Teile Riboflavin-Phosphatnatrium in 40 Teilen Wasser gelöst und zum Teil suspendiert. Die beiden Teilansätze werden vereinigt und gut vermischt. Das stabilisierte Einbettungsprodukt wird in an sich bekannter Weise durch Sprühtrocknung hergestellt. Man kann das erhaltene Reaktionsgemisch auch auf Kühlwalzen nach Abdampfen des Lösungsmittels erstarren lassen. In diesem Fall wird das erhaltene Produkt vor seiner Weiterverwendung noch granuliert.

**Beispiel 2**

50 Teile Nifedipin werden in 50 Teilen Chloroform unter gelindem Erwärmen gelöst. 43 Teile geschmolzenes Polyethylenglykol 10000 werden zugesetzt. Getrennt werden 2 Teile Riboflavin, löslich in der warmen Lösung von 5 Teilen Cremophor RH 60® und 15 Teilen Wasser, homogen dispergiert. Die beiden Teilansätze werden vereinigt und, wie in Beispiel 1 angegeben, zu dem Einbettungsprodukt weiterverarbeitet.

**Beispiel 3**

50 Teile Nifedipin werden in der warmen Lösung von 30 Teilen Tagat RH 40 ® und 49 Teilen Polyethylenglykol 20000 in 75 bis 90 Teilen Aceton gelöst. Getrennt wird 1 Teil Riboflavin, löslich in 5 Teilen Wasser, teils gelöst, teils suspendiert. Die beiden Teilansätze werden vereinigt,und, wie in Beispiel 1 beschrieben, wird das stabilisierte Einbettungsprodukt hergestellt.

**Beispiel 4**

50 Teile Nifedipin werden in 100 Teilen Cyclohexanol unter gelindem Erwärmen gelöst und 27 Teile geschmolzenes Polyethylenglykol 4000 werden zugesetzt. Getrennt werden 3 Teile Riboflavin-Phosphatnatrium in der warmen Lösung von 20 Teilen Polyethoxy-50-stearat und 30 Teilen Wasser weitgehend gelöst. Die beiden Teilansätze werden vereinigt und, wie in Beispiel 1 angegeben, zu dem Einbettungsprodukt weiterverarbeitet.

**Beispiel 5**

Zur Herstellung von Weichgelatinekapseln werden, berechnet pro Kapsel, 20 mg des gemäß den Beispielen 1 bis 4 hergestellten, eingebetteten Nifedipins in 358,9 mg Polyethylenglykol 300 oder 400 gelöst, und anschließend werden 0,6 mg Pfefferminzöl sowie eine Lösung von 0,5 mg Saccharinnatrium in 5 mg Wasser zugesetzt.

Diese Lösung wird in bekannter Weise in Weichgelatinekapsein abgefüllt, so daß jede Kapsel 10 mg reines Nifedipin enthält.

**Beispiel 6**

Zur Herstellung von Filmtabletten werden pro Tablette 40 mg des eingebetteten Nifedipins, hergestellt gemäß den Beispielen 1 bis 4, mit 50 mg Tablettose *, 40 mg mikrokristalliner Cellulose, 19 mg Mais- oder Weizenstärke und 1 mg Magnesiumstearat homogen vermischt und durch ein Sieb mit 0,1 bis 0,15 mm lichter Maschenweite geschlagen. Diese Mischung wird in an sich bekannter Weise zu Tabletten mit einem Bruttogewicht von 150 mg und einem Gehalt von 20 mg reinem Nifedipin komprimiert. Die Tablette wird anschließend mit einer zum Lichtschutz mit Eisenoxidpigmenten eingefärbten Filmumhüllung überzogen.

**Beispiel 7**

Zur Herstellung einer Mischung für Hartgelatinekapseln werden, berechnet pro Kapsel, 20 mg des erfindungsgemäß eingebetteten Nifedipins, hergestellt gemäß einem der Beispiele 1 bis 4, mit 30 mg mikrokristalliner Cellulose, 25 mg Dicalciumphosphat, 20 mg Stärke und 5 mg Talkum gemischt und gesiebt. Diese Mischung wird in an sich bekannter Weise in Hartgelatine-Steckkapseln mit dem vorgeschriebenen Wirkstoffgehalt abgefüllt.

**Patentansprüche**
**Patentansprüche für folgende Vertragsstaaten : AT, BE, CH, DE, FR, GB, GR, IT, LI, LU, NL, SE**

1. Gegenüber Lichteinfluß stabilisiertes Nifedipin-Konzentrat, dadurch **gekennzeichnet,** daß es Nifedipin, Polyethylenglykol, 0,5 bis 20 Gew.-% Vitamin $B_2$, bezogen auf das Nifedipin, mindestens ein Tensid und gegebenenfalls übliche Trägerstoffe enthält.

2. Nifedipin-Konzentrat nach Anspruch 1, dadurch **gekennzeichnet,** daß das Verhältnis Nifedipin:Polyethylenglykol im Bereich zwischen 2:1 und 1:3 liegt.

3. Nifedipin-Konzentrat nach Anspruch 1 oder 2, dadurch **gekennzeichnet,** daß es 2 bis 50 Teile Tensid, bezogen auf 50 Teile Nifedipin, enthält.

4. Nifedipin-Konzentrat nach Anspruch 1, 2 oder 3, dadurch **gekennzeichnet,** daß es als Tensid Ethoxy-Propoxy-Copolymere, Polyoxyethylen-Fettsäureester, Polyoxyethylen-Fettalkoholether oder ethoxylierte,

* = (optimal tablettierbare Lactose)

hydrierte Ricinusölprodukte oder Gemische dieser Verbindungen enthält.

5. Verfahren zur Herstellung der gegenüber Lichteinfluß stabilisierten Nifedipin-Konzentrate gemäß einem der Ansprüche 1 bis 4, dadurch **gekennzeichnet,** daß man Nifedipin, das Polyethylenglykol und gegebenenfalls das Tensid in einem organischen Lösungsmittel, gegebenenfalls unter Erwärmen, löst und zu der nifedipinhaltigen Lösung eine wäßrige Lösung oder Suspension von Vitamin $B_2$ und gegebenenfalls Tensid zugibt, wobei mindestens eine der beiden Lösungen bzw. die Suspension Tensid enthält, die beiden Lösungen mischt und in an sich bekannter Weise eindampft und das stabilisierte Einbettungsprodukt gewinnt und gegebenenfalls granuliert.

6. Verfahren nach Anspruch 5, dadurch **gekennzeichnet,** daß man als organisches Lösungsmittel Aceton, chlorierte Kohlenwasserstoffe oder Toluol verwendet.

7. Verfahren nach Anspruch 5 oder 6, dadurch **gekennzeichnet,** daß man Nifedipin und das Polyethylenglykol in einem Verhältnis zwischen 2:1 und 1:1 verwendet und daß man 0,5 bis 20 Gew.-% Vitamin $B_2$, bezogen auf das Nifedipin, verwendet.

8. Verfahren nach einem der Ansprüche 5, 6 oder 7, dadurch **gekennzeichnet,** daß das Tensid in einer Menge von 2 bis 50 Teilen, bezogen auf das Nifedipin, verwendet wird.

9. Verwendung von dem Nifedipin-Konzentrat nach einem der Ansprüche 1 bis 4 zur Herstellung von Arzneimittelzubereitungen.

**Patentansprüche für folgenden Vertragsstaat : ES**

1. Verfahren zur Herstellung eines gegenüber Lichteinfluß stabilisierten Nifedipin-Konzentrats, dadurch **gekennzeichnet,** daß man Nifedipin, das Polyethylenglykol und gegebenenfalls ein Tensid in einem organischen Lösungsmittel, gegebenenfalls unter Erwärmen, löst und zu der nifedipinhaltigen Lösung eine wäßrige Lösung oder Suspension von Vitamin $B_2$ und gegebenenfalls Tensid zugibt, wobei mindestens eine der beiden Lösungen bzw. die Suspension Tensid enthält, die beiden Lösungen mischt und in an sich bekannter Weise eindampft und das stabilisierte Endprodukt gewinnt und gegebenenfalls granuliert.

2. Verfahren nach Anspruch 1, dadurch **gekennzeichnet,** daß man als organisches Lösungsmittel Aceton, chlorierte Kohlenwasserstoffe oder Toluol verwendet.

3. Verfahren nach Anspruch 1 oder 2, dadurch **gekennzeichnet,** daß man Nifedipin und das Polyethylenglykol in einem Verhältnis zwischen 2:1 und 1:1 verwendet und daß man 0,5 bis 20 Gew.-% Vitamin $B_2$, bezogen auf das Nifedipin, verwendet.

4. Verfahren nach einem der Ansprüche 1, 2 oder 3, dadurch **gekennzeichnet,** daß man als Tensid Ethoxy-Propoxy-Copolymere, Polyoxyethylen-Fettsäureester, Polyoxyethylen-Fettalkoholether oder ethoxylierte hydrierte Ricinusölprodukte oder Gemische dieser Verbindungen verwendet.

5. Verfahren nach einem der Ansprüche 1 bis 4, dadurch **gekennzeichnet,** daß das Tensid in einer Menge von 2 bis 50 Teilen, bezogen auf das Nifedipin, verwendet wird.

**Claims**
**Claims for the following Contracting States : AT, BE, CH, DE, FR, GB, GR, IT, LI, LU, NL, SE**

1. Nifedipine concentrate stabilized against the influence of light, **wherein** Nifedipine, polyethylene glycol, 0.5 to 20 per sent by weight of Vitamine $B_2$, as related to the Nifedipine, at least one tenside, and common carriers as required are contained.

2. Nifedipine concentrate as per claim 1, **wherein** the Nifedipine: polyethelene glycol ratio ranges from 2 to 1 to 1 to 3.

3. Nifedipine concentrate as per claim 1 or 2, **wherein** 2 to 50 parts of a tenside, as related to 50 parts of Nifedipine is contained.

4. Nifedipine concentrate as per claims 1, 2 or 3, **wherein** a tenside is contained, such tenside being either ethoxy-propoxy- copolymeres, polyoxyethylene fatty acid esters, polyoxyethylene fatty alcohol ethers, or ethoxylated hydrated castor oil products, or mixtures of such compounds.

5. Method of production of a Nifedipine concentrate stabilized against the influence of light according to claims 1 to 4, **wherein** Nifedipine, polyethylene glycol and the tenside as required are dissolved in an organic solvent, warming this solution if necessary. To the Nifedipine solution thus obtained is added an aqueous solution, or suspension of vitamin $B_2$, and a tenside as required, such tenside to be contained in at least one of the two solutions or the suspension respectively. Both solutions are then combined and reduced by evaporation in known manner and the resulting embedded product is ultimately granulated.

6. Method according to claim 5, **wherein** acetone, chlorinated hydrocarbons, or toluene are used as organic solvents.

7. Method according to claim 5 or 6, **wherein** Nifedipine and polyethylene glycol are employed at a ratio of 2 to 1 and 1 to 1, and 0.5 to 20 percent by weight of vitamine $B_2$, as related to the Nifedipine is employed.

8. Method according to one of claims 5, 6 or 7, **wherein** the tenside is used in a quantity of 2 to 50 parts as related to the Nifedipine.

9. Use of the Nifedipine concentrate in accordance with one of claims 1 to 4 in formulating medicament preparations.

**Claims for the following Contracting State : ES**

1. Method for the production of a Nifedipine concentrate stabilized against the influence of light, **wherein** Nifedipine, polyehtylene glycol and a tenside as required are dissolved , warming the composition if required, in an organic solvent, adding to the Nifedipine-containing solution thus obtained, an aqueous solution or suspension of vitamine $B_2$ and a tenside if required, wherein at least one of the two solutions or the suspension respectively contains a tenside, combining the two solutions and reducing the compound by evaporation in known manner, thereby obtaining the stabilized final product, which may then be granulated as desired.

2. Method according to claim 1, **wherein** the organic solvent employed is either acetone, chlorinated hydrocarbons, or toluene.

3. Method according to claims 1 or 2, **wherein** Nifedipine and the polyehtylene glycol are employed at ratios between 2:1 and 1:1, and that 0.5 to 20 per cent by weight of vitamin $B_2$, as related to the Nifedipine, is employed.

4. Method according to one of claims 1, 2 or 3, **wherein** a tenside is contained, such tenside being either ethoxy-propoxy- copolymeres, polyoxyethylene fatty acid esters, polyoxyethylene fatty alcohol ethers, or ethoxylated, hydrated caster oil products, or mixtures of such compounds.

5. Method according to one of claims 1 to 4, **wherein** the tenside is used in a quantity of 2 to 50 parts, as related to the Nifedipine.

**Revendications**
**Revendications pour les Etats contractants suivants : AT, BE, CH, DE, FR, GB, GR, IT, LI, LU, NL, SE**

1. Concentré de Nifédipine stabilisé contre l'action de la lumière, caractérisé en ce qu'il contient de la Nifédipine, du polyéthylèneglycol, 0,5 à 20 % en poids de vitamine $B_2$, par rapport à la Nifédipine, au moins un tensioactif et éventuellement des matières de support habituelles.

**2.** Concentré de Nifédipine selon la revendication 1, caractérisé en ce que le rapport Nifédipine/polyéthylèneglycol est compris entre 2:1 et 1:3.

**3.** Concentré de Nifédipine selon la revendication 1 ou 2, caractérisé en ce qu'il contient de 2 à 50 parties de tensioactif pour 50 parties de Nifédipine.

**4.** Concentré de Nifédipine selon la revendication 1, 2 ou 3, caractérisé en ce qu'il contient comme tensioactifs des éthoxy-propoxy-copolymères, des esters d'acides gras de polyoxyéthylène, des éthers d'alcool gras de polyoxyéthylène ou des produits d'éthoxylation de l'huile de ricin hydrogénés ou des mélanges de ces substances.

**5.** Procédé pour la fabrication des concentrés de Nifédipine stabilisés contre l'action de la lumière selon l'une des revendications 1 à 4, caractérisé en ce que l'on dissout la Nifédipine, le polyéthylèneglycol et éventuellement le tensioactif dans un solvant organique, éventuellement en chauffant, et on ajoute à la solution contenant la Nifédipine une solution ou suspension aqueuse de vitamine $B_2$ et éventuellement le tensioactif, l'une au moins des deux solutions ou la suspension contenant le tensioactif, on mélange les deux solutions et on les évapore de manière connue et on récupère le produit d'enrobage stabilisé et éventuellement on le granule.

**6.** Procédé selon la revendication 5, caractérisé en ce que l'on utilise comme solvant organique l'acétone, les hydrocarbures chlorés ou le toluène.

**7.** Procédé selon la revendication 5 ou 6, caractérisé en ce que l'on utilise la Nifédipine et le polyéthylèneglycol dans un rapport compris entre 2:1 et 1:1 et on utilise 0,5 à 20 % en poids de vitamine $B_2$ par rapport à la Nifédipine.

**8.** Procédé selon l'une quelconque des revendications 5, 6 ou 7, caractérisé en ce que le tensioactif est utilisé en quantité de 2 à 50 parties par rapport à la Nifédipine.

**9.** Utilisation du concentré de Nifédipine selon l'une des revendications 1 à 4 pour la fabrication de compositions de médicaments.

**Revendications pour l'Etat Contractant suivant : ES.**

**1.** Procédé pour la fabrication d'un concentré de Nifédipine stabilisé contre l'action de la lumière, caractérisé en ce que l'on dissout la Nifédipine, le polyéthylèneglycol et éventuellement le tensioactif dans un solvant organique, éventuellement en chauffant, et on ajoute à la solution contenant la Nifédipine une solution ou suspension aqueuse de vitamine $B_2$ et éventuellement le tensioactif, l'une au moins des deux solutions ou la suspension contenant le tensioactif, on mélange les deux solutions et on les évapore de manière connue et on récupère le produit d'enrobage stabilisé et éventuellement on le granule.

**2.** Procédé selon la revendication 1, caractérisé en ce que l'on utilise comme solvant organique l'acétone, les hydrocarbures chlorés ou le toluène.

**3.** Procédé selon la revendication 1 ou 2, caractérisé en ce que l'on utilise la Nifédipine et le polyéthylèneglycol dans un rapport compris entre 2:1 et 1:1 et on utilise 0,5 à 20 % en poids de vitamine $B_2$ par rapport à la Nifédipine.

**4.** Procédé selon la revendication 1, 2 ou 3, caractérisé en ce qu'il contient comme tensioactifs des éthoxy-propoxy-copolymères, des esters d'acides gras de polyoxyéthylène, des éthers d'alcools gras de polyoxyéthylène ou des produits d'éthoxylation de l'huile de ricin hydrogénés ou des mélanges de ces substances.

**5.** Procédé selon l'une des revendications 1 à 4, caractérisé en ce que le tensioactif est utilisé en quantité de 2 à 50 parties par rapport à la Nifédipine.

FIGUR 1:     Nifedipin-Einbettung mit Riboflavin

⊗   Anfangskurve

▲   nach 3 Tagen am Licht

280 nm     350 nm

FIGUR 2:     Nifedipin-Einbettung <u>ohne</u> Riboflavin

⊗   Anfangskurve

▲   nach 3 Tagen am
    Licht

280 nm        350 nm

EP 0 272 336 B1